(19) Europäisches Patentamt
European Patent Office
Office européen des brevets

(11) **EP 4 506 947 A1**

(12) **EUROPEAN PATENT APPLICATION**

(43) Date of publication:
**12.02.2025 Bulletin 2025/07**

(21) Application number: **23306367.6**

(22) Date of filing: **11.08.2023**

(51) International Patent Classification (IPC):
**G16B 5/00** $^{(2019.01)}$  **G16B 25/00** $^{(2019.01)}$

(52) Cooperative Patent Classification (CPC):
**G16B 25/00; G16B 5/00**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Vidium Solutions**
**69457 Lyon 06 (FR)**

(72) Inventor: **The designation of the inventor has not
yet been filed**

(74) Representative: **Abel & Imray LLP**
**Westpoint Building**
**James Street West**
**Bath BA1 2DA (GB)**

(54) **COMPUTER-IMPLEMENTED METHODS OF CONSTRUCTING GENE REGULATORY
NETWORKS**

(57) A computer-implemented method of constructing a gene regulatory network for a set of cells. A gene
regulatory network is a network showing interactions
between a plurality of genes. Data including a set of gene
quantity values is received, each gene quantity value
being associated with a combination of a cell of the set
of cells and a gene of the cell. Each gene quantity value is
indicative of a measurement of a gene quantity for the
gene, a gene quantity being a quantity of a product
resulting from the expression of the gene. A plurality of
sets of the gene quantity values are determined, and for
each set, a time value is determined from the gene
quantity values. For each of a plurality of genes, the level
of the gene quantity value for the gene over time is
determined from the determined times of the sets. A
wave transition time for the gene quantity value for each
gene is then determined from the determined the level of
the gene quantity value over time. A wave transition time
is indicative of the time at which a gene quantity value
passes from one steady state to another steady state. A
set of interactions between the genes is then determined
using the wave transition times, and a gene regulatory
network is constructed from the determined set of inter-
actions.

Fig. 1

EP 4 506 947 A1

**Description**

Field of the Invention

The present invention concerns computer-implemented methods of constructing gene regulatory networks for cells. More particularly, but not exclusively, the present invention concerns constructing gene regulatory networks by determining the times at which changes in the behaviour of genes of cells occur, and by determining the types of interaction between genes that are occurring at different times.

Background of the Invention

Gene regulatory networks (GRNs) are networks showing interactions between genes of cells over time, so describing the behaviour of the cells in terms of their genes. Understanding the behaviour of cells in terms of their genes can have many valuable applications, such as enabling treatments for genetic conditions to be identified, to give just one of many examples.

The genes of a cell can be expressed at different times and to different extents, depending on environmental conditions. These environmental conditions will themselves depend on the expression of other genes. When a gene is expressed, proteins associated with the expression of the gene will be produced. These proteins can promote or inhibit the expression of other genes (or indeed the gene producing the protein), which will in turn produce further proteins, which promote or inhibit the expression of further genes. Over time, the proteins produced by expressed genes will degrade, with different proteins degrading at different rates.

As a consequence of the above, the interactions between the genes of cells over time can be very complicated, with the expression of genes being promoted and inhibited at different levels at different times. In particular, the reason for the behaviour of a gene at one time in the life of a cell may be different to the reason for the same behaviour at another time.

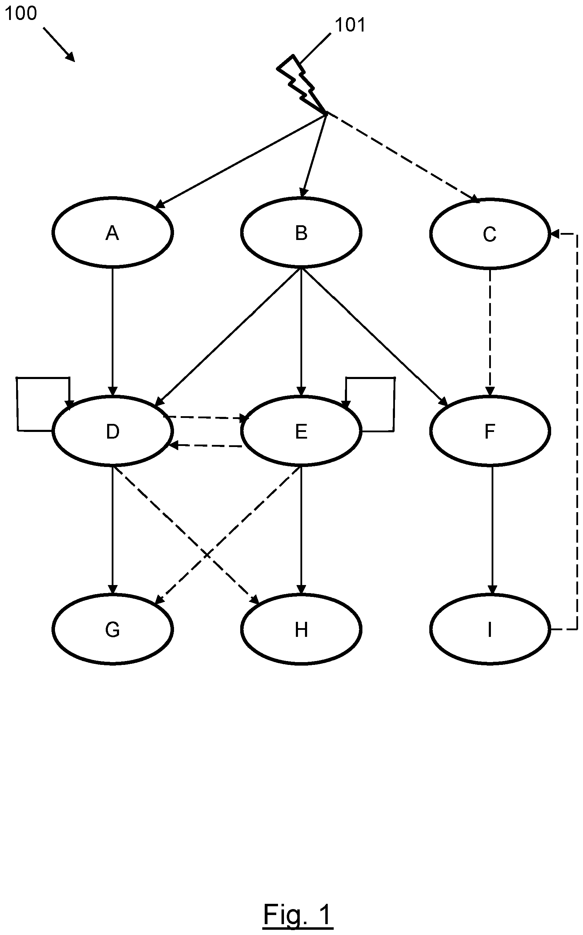

Figure 1 shows an example of a GRN 100, which is network of nodes and arrows between the nodes. The node 101, shown as a lightning symbol, represents an initial stimulus, such as the presence of a drug or stress on the cell, while the nodes A to I represent genes. A solid arrow from a first gene to a second gene represents the promotion of the second gene by the first gene (or the stimulus), while a dashed arrow represents the inhibition of the second gene by the first gene (or the stimulus). Thus, it can be seen that the stimulus initially promotes the expression of the genes A and B, and inhibits the expression of the gene C. The gene A in turn promotes the expression of the gene D; the gene B promotes the expression of the genes D, E and F, while the gene C inhibits the expression of the gene F. The gene D promotes its own expression, and that of the gene G, while inhibiting the expression of the genes E and H. Somewhat similarly, the gene E promotes its own expression, and that of the gene H, while inhibiting the expression of the genes D and G. Finally, the gene F promotes the expression of the gene I, which in turn inhibits the expression of the gene C.

It will be appreciated that the promoting and inhibiting effects of the genes, which may be on multiple other genes, and may result in looping behaviour (for the loop from gene C to F to I and then back to C), can cause extremely complicated behaviour over time, while is the result of genes affecting other genes in ways which it is very difficult to determine merely from observing the behaviour of the genes over time. This is particularly the case because it is not possible to directly measure the level of expression of a gene at a particular time, but rather only the amounts of the products that result from the gene being expressed, e.g. the amount of RNA or protein resulting from the gene being expressed that is present at a particular time.

A further particular problem that arises is that many methods for measuring the amounts of products produced by genes result in the destruction of the cell. This means that using such methods, it is not possible to observe the behaviour of the genes of a cell over a period time. Rather, it is only possible to get a snapshot of the behaviour of the genes of a call at a single point in time.

All of the above means that it can be very difficult to correctly determine the GRN describing the behaviour of the genes of a cell using the data that can be obtained by measuring the behaviour of cells.

The present invention seeks to solve or mitigate some or all of the above-mentioned problems. Additional or alternatively, the present invention seeks to provide improved computer-implemented methods of constructing gene regulatory networks for sets of cells.

Summary of the Invention

In accordance with a first aspect of the invention there is provided a computer-implemented method of constructing a gene regulatory network for a set of cells, wherein a gene regulatory network is a network showing interactions between a plurality of genes, the method comprising the steps of:

   receiving data including a set of gene quantity values, wherein each gene quantity value is associated with a combination of a cell of the set of cells and a gene of the cell, and wherein each gene quantity value is indicative of a measurement of a gene quantity for the gene, wherein a gene quantity is a quantity of a product resulting from the expression of the gene;

determining a plurality of sets of the gene quantity values, wherein each gene quantity value of a set of gene quantity values is associated with a different gene;

for each of the plurality of sets of gene quantity values, determining a time value for the set of gene quantity values from the gene quantity values of the set of gene quantity values;

for each of a plurality of genes, determining the level of the gene quantity value for the gene over time from the determined times of the sets of gene quantity values containing the gene quantity value;

for each of a plurality of genes, determining a wave transition time for the gene quantity value for the gene from the determined the level of the gene quantity value for the gene over time, wherein the wave transition time is indicative of the time at which a gene quantity value passes from one steady state to another steady state;

determining a set of interactions between the genes using the wave transition times for the plurality of genes; and

constructing a gene regulatory network from the determined set of interactions between the genes.

By determining a plurality of suitable sets of gene quantity values, a collection of sets that indicate the behaviour of a cell at different times is obtained. These can then be used to determine how the gene quantity values change over time, and from those changing values, wave transition times, i.e. times at which the gene quantity values change between steady states, can be determined. These wave transition times can then be used to determine possible interactions between the genes, i.e. interactions that could have resulted in the determined wave transition times. This is possible because, while for any single cell, only a snapshot of its behaviour at a single moment in time is measure, the "ergodic assumption" is used, under which measuring multiple cells at one time is considered to be equivalent to measuring a single cell at multiple times. In this way, the received data for the multiple cells can be used to determine how a single cell would behave over time. The determined set of interactions can then be used to construct the gene regulatory network, by combining the interactions as appropriate, particularly including, where more than one interaction could have resulted in the determined wave transition times, selecting the appropriate interaction.

A gene quantity of the gene quantities may be one of:

an RNA count associated with the expression of the gene; and

a protein count associated with the expression of the gene. However, any suitable gene quantities resulting from the expression of genes may be used.

The time value for each set of gene quantity values may be determined by:

determining a genetic distance value indicative of the difference between the gene quantity values of the set of gene quantity values and corresponding gene quantity values of a reference cell;

determining an RNA velocity value indicative of the rate of change of the gene quantity values of the set of gene quantity values in a reference cell; and

determining the time value from the genetic distance value and the RNA velocity value. The time value may be the genetic distance value divided by the RNA velocity value.

The gene quantity values may be indicative of a measurement a spliced RNA count and an unspliced RNA count for the gene, and wherein the RNA velocity is calculated using the spliced RNA count and the unspliced RNA count. In this case, the method may further comprise the step of receiving, for each combination of a cell and a gene, an RNA degradation rate, where the RNA degradation rate is indicative of the degradation rate of the spliced RNA, and wherein the RNA velocity is calculated using the spliced RNA count, the unspliced RNA count and the RNA degradation rate.

The method may further comprise the step of obtaining one or more genetic trajectories for cells of the set of cells, where a genetic trajectory for a cell is indicative of changes in steady states of the expression of a set of genes of the cell over a period of time, and wherein one or more wave transition times are determined using the one or more genetic trajectories. The genetic trajectories are indicative of changes in gene expression over time, but without any indication of the interaction between the genes that results in the gene expressions. The genetic trajectories may be obtained from the received data, for example from transcriptomes of cells. Alternatively, they may be obtained with input from a user, for example using other analysis tools on the data. The genetic trajectories may be used to identify genes to focus on to construct the GRN.

Particularly, the method may further comprise the step of determining a time ordering for a plurality of cells of the set of cells using the genetic quantity values for the cell and their associated genes, where the ordering is in accordance with the ordering of the expressions of the genes in the one or more genetic trajectories. Each cell will have particular set of genetic quantity values, with corresponding levels of expressions of genes, and so as the genetic trajectories give an ordering for different levels of expressions of genes, they can be used to order the cells. The time ordering can indicate an order in which the cell states occur in time, but without indicating specific times, i.e. not indicting how soon one cell state happens after another, but rather only the time order in which they happen.

The method may further comprise the steps of:

determining a cell group, wherein a cell group is a subset of the plurality of cells, and wherein each cell in the cell group is adjacent to at least one other cell in the time ordering for the plurality of cells;

identifying one or more transient genes of the cell group, wherein a transient gene is a gene whose gene quantity value passes from one steady state to another steady state in the cells of the cell group;

determining an overall genetic distance value for the one or more transient genes of the cell group;

determining an overall RNA velocity value for the one or more transient genes of the cell group;

determining an overall time value for the cell group from the overall genetic distance value and the overall RNA velocity value; and

using the overall time value for the cell group to determine the wave transition times. The overall time value may be the overall genetic distance value divided by the overall RNA velocity value. The overall genetic distance value may be the average of genetic distance values for multiple transient genes of the cell group. The overall RNA velocity value may be the average of RNA velocity values for multiple transient genes of the cell group. An overall time value for each of a plurality of cell groups may be determined, wherein the overall genetic distance value for a first cell group is indicative of the difference between the gene quantity values of the cells of the first cell group and the corresponding gene quantity values of a second cell group.

At least one interaction of the set of interactions between the genes may be included in the set of interactions if the interaction is between a first gene and a second gene, and the difference between a wave transition time for the first gene and between a wave transition time for the second gene is below a threshold. The interaction may also be between other genes, and not only the first gene and the second gene. The threshold may be determined using the protein degradation rate of first gene and RNA degradation rate of the second gene.

In accordance with a second aspect of the invention there is provided a computer-implemented method of constructing a gene regulatory network for a set of cells, wherein a gene regulatory network is a network showing interactions between a plurality of genes, the method comprising the steps of:

receiving data including a set of gene quantity values, wherein each gene quantity value is associated with a combination of a cell of the set of cells and a gene of the cell, and wherein each gene quantity value is indicative of a measurement of a gene quantity for the gene, wherein a gene quantity is a quantity of a product resulting from the expression of the gene;

determining from the set of gene quantity values a plurality of gene interactions, wherein a gene interaction comprises one of more regulators which are indicative of a stimulus or a gene of the set of genes, one or more targets which are indicative of genes of the set of genes, and a set of relationships which are indicative of promotion or inhibition of a target of the gene interaction by a regulator of the gene interaction;

determining a network of sets of gene interactions, wherein each gene interaction of a set of gene interactions shares at least one regulator and at least one target, and wherein a first set of gene interactions is dependent on a second set of gene interactions only if a shared regulator of the first gene interaction is a shared target of the second gene interaction;

constructing the gene regulatory network from the network of sets of gene interactions, by selecting a gene interaction of each set of gene interactions, and combining the selected gene interactions by, for each selected gene interaction, and for each selected gene interaction on which the first selected gene interaction is dependent, matching each regulator of the first selected gene interaction with a corresponding target of a selected gene interaction on which it is dependent.

In this way, sets of gene interactions, which represent possible interactions between genes that could have resulted in observed behaviour of the genes (as determined from the gene quantity values), are put into a network. As in the first aspect, this is possible because the ergodic assumption is used, and so the received data for the multiple cells can be determine how a single cell would behave over time. A GRN can then be constructed, by selecting an appropriate gene interaction from each set, and combining the selected gene interactions together.

A second gene interaction will not always be dependent upon a first gene interaction when a source gene of the second gene interaction is a target gene of the first interaction, but where there is a dependency, this will necessarily be the case (otherwise the dependency would not be valid).

The method may further comprise the steps of:

calculating a quality score for the constructed gene regulatory network; comparing the quality score with a calculated quality score for a previously constructed gene regulatory network; and

constructing a further gene regulatory network from the network of sets of gene interactions, wherein the gene interactions are selected from each set of gene interactions based on the calculated quality scores. The method may be repeated to find gene regulatory networks constructed from gene interactions with successively higher quality scores. The method may continue to be repeated until the calculated quality scores are no longer increasing, or increase less than a threshold, for example.

The method may further comprise the step of storing the quality score for the constructed gene regulatory network against the gene interactions selected to construct the gene regulatory network. In this case, the gene interactions selected from each set of gene interactions are selected using the stored quality scores using a Monte Carlo tree search. In this way, a quality score for a gene interaction is recorded, indicating the quality of the GRN it was most recently used to construct. These quality scores can then be used to determine which gene interactions to try to construct a new GRN. The use of a Monte Carlo tree search has surprisingly been found to be particularly effective at finding GRNs of high quality.

The quality score for a gene interaction may be determined by:

simulating the behaviour of the genes of the gene interaction; and

determining the behaviour of the genes of the gene interaction from the received data;

wherein the quality score is indicative of how close the simulated behaviour of the genes of the gene interaction is to their determined behaviour. The closeness of the simulated behaviour and the determined behaviour may be calculated using a distance metric. The distance metric may be the Kantorovitch distance. The wave transition times for the genes may be used to determine the quality score.

The methods described above may be combined in any desired manner. However, it will be appreciated that while the ways in which wave transition times are determined are particularly applicable to the ways in which GRNs are constructed from networks of sets of dependent gene interactions sharing regulators and targets (such networks particularly including the generic networks discussed below), the former is equally applicable where the latter is done using any other method, and *vice versa.*

The methods may further comprise the step of performing measurements on the set of cells to determine the set of gene quantity values for the set of cells.

In accordance with a third aspect of the invention there is provided a computer program product comprising machine-readable instructions that, when executed on a computing device comprising a processor and memory, cause the computing device to perform the steps of any of the methods described above.

It will of course be appreciated that features described in relation to one aspect of the present invention may be incorporated into other aspects of the present invention. For example, the method of the invention may incorporate any of the features described with reference to the apparatus of the invention and *vice versa.*

Description of the Drawings

Embodiments of the present invention will now be described by way of example only with reference to the accompanying schematic drawings of which:

Figure 1 is a diagram showing an exemplary GRN;

Figure 2 is a flowchart showing the steps of a method of constructing a GRN in accordance with an embodiment of the invention;

Figures 3a to 3e are diagrams showing relationships between different GRN structures, genetic trajectories and waves;

Figures 4a and 4b are diagrams of exemplary generic networks;

Figures 4c is a diagram showing an exemplary GRN;

Figure 5 is a flowchart showing in more detail the identification of wave transition times;

Figure 6 is a flowchart showing in more detail the calculation absolute group times for cell groups;

Figure 7 is a diagrams showing the determination of a genetic interaction from wave transition times;

Figure 8 is a flowchart showing in more detail the construction of GRNs from generic networks;

Figure 9a is a diagram showing a generic network;

Figure 9b is a diagram showing a Monte Carlo tree;

Figure 9c is a diagram showing successive wave calibration jobs performed in order to construct a GRN;

Figure 9d is a diagram showing a constructed GRN; and

Figure 10 is a schematic diagram of a computing device in accordance with embodiments of the invention.

Detailed Description

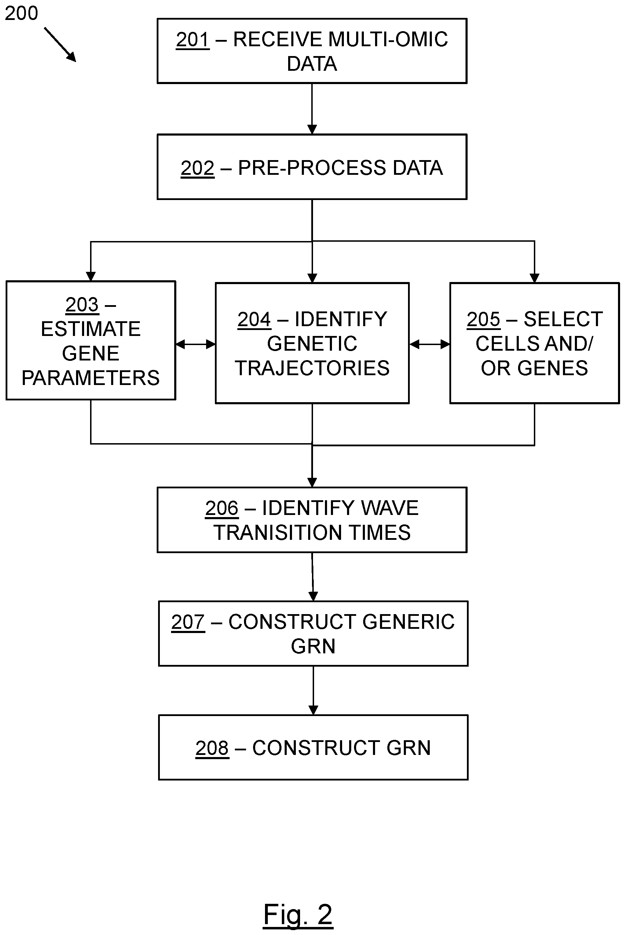

A computer-implemented method of determining a gene regulatory network for a set of cells is now described, with reference to Figures 2 to 10.

Figure 2 is a flowchart showing, at a high level, the overall method of the present embodiment. In a first step, for a set of cells, multi-omic data, i.e. data relating to the genetic behaviours of the cells (such as RNA, protein, epigenetic data and the like), is received (step 201). This multi-omic data includes in particular data regarding the products of the expression of the genes of the cells of the set of cells. The set of cells will include many cells of the same type, but at different stages in their lives. The set of cells may also include cells of related types, for example when a cell changes type over its life, or merely because different types of cell were obtained together and analysed as part of the same set. By analysing this set of cells, the data will contain data regarding the state of a cell of a particular type at many different times in its life, but without it being necessary to measure a single cell at different times.

It will be appreciated that multi-omic data received may comprise a data set that contains different cell types, depending on the sample used to generate the data set. For example, all cells obtained from a subject may be used to create the data set, for example all cells from an organ of a patient. Alternatively, the desired cells may be extracted prior to being used to generate the data set. In the present embodiment, cells are studied by cell types. It is supposed that cells do not belong to the same cell type if there is a discontinuity in the UMAP (uniform manifold approximation and projection) for the cells, and in this way the cells can be clustered by cell type, using conventional methods well known to the skilled person. If there is a continuum between two cell types, the cells are considered to be of a unique cell type. Thus, groups of cells belonging to a continuum are always analysed separately.

The data is then pre-processed (step 202), using any suitable techniques, of which various will be well-known to the skilled person. The pre-processing of the data cleans and normalises it, and converts it into a form in which it can be used in the later steps of the method.

Next, using the pre-processed data gene parameters are estimated (step 203), genetic trajectories are identified (step 204), and cells and/or genes to be used in the method are selected (step 205), each of which is discussed in more detail below. These steps can be performed iteratively, with the results of one step allowing refinement of an iteration of another step, and so are shown in parallel and in dependence on each other in the flowchart of Figure 2.

The results from the above steps are then used to identify wave transition times (step 206), which correspond to times at which the product of a gene expression moves from one steady state to another, as discussed in detail below.

The wave transition times are then used to construct a generic network (step 207). A generic network is a generalised version of a GRN, in which the nodes of the network can contain different possible gene interactions. The generic network is then used to construct a GRN (step 208). The construction of the generic network, and the GRN, are discussed in detail below. (In fact, multiple GRNs may be constructed, to allow an optimal GRN to be found, again as discussed.)

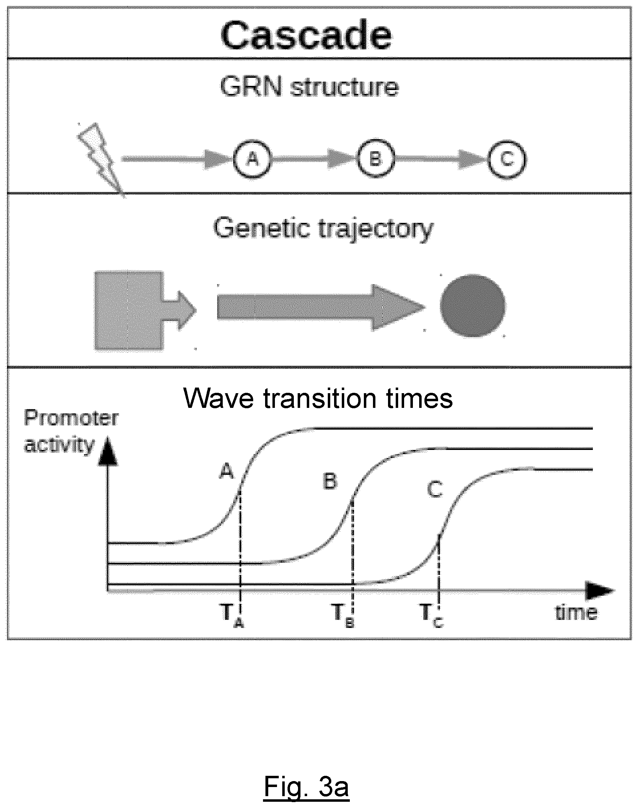

As mentioned above, the method of the present embodiment involves the construction of a GRN from generic network, both of which represent interactions between genes, and are formed of combinations of different types of GRN structures. These GRN structures are obtained from the received data using gene trajectories identified from the data; a gene trajectory represents the overall changes in the expression of certain genes of a cell over a period of time, but without any indication of the particular interactions between the genes that causes the changes to occur. In combination with the gene trajectories, waves in the amount of products produced by the expressions of genes that are present are used; thus, the waves are representative of expressions of the genes over time. Figure 3a to 3e are diagrams showing relationships between certain different GRN structures, genetic trajectories, and waves.

Figure 3a shows a cascade GRN structure, in which stimulus promotes the expression of a gene A, as shown by the green arrow, which in turn promotes the expression of a gene B, which in turn promotes the expression of a gene C.

The genetic trajectory corresponding to this GRN structure is that the genes A, B and C are initially in a state in which none of the genes are expressed (the green square), and end in a state in which all of the genes are expressed (the red circle).

The wave transition times are then shown in a graph showing the change in promoter activity for each of the genes over time. Initially, the promoter activity for each of the genes A, B and C is at a low steady state. Due to the stimulus, the promoter activity for the gene A rises to high steady state. The wave transition time $T_A$ is indicative of the time of the change between the steady states, and can be taken to be the mean time of the change between states. As the expression of gene A promotes the expression of gene B, the promoter activity for gene B next moves from a low steady state to a high steady state, with wave transition time $T_B$. Finally, the promoter activity for gene C next moves from a low steady state to a high steady state, with wave transition time Tc, giving a final state in which all genes are expressed, and correspondingly all promoter activities for the genes are at the high steady state.

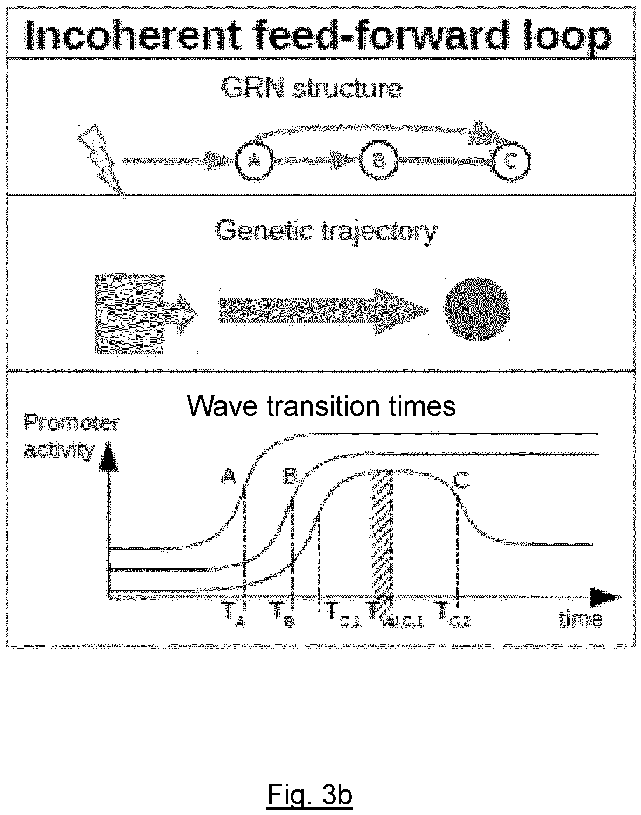

Figure 3b shows an incoherent feed-forward loop GRN structure. In this GRN structure, a stimulus promotes the expression of a gene A, and the expression of gene A promotes the expression of a gene B, and also a gene C. The expression of gene B then inhibits the expression of gene C, as shown by the red arrow.

The genetic trajectory corresponding to this GRN structure is that the genes A, B and C are initially in a state in which none of the genes are expressed (the green square), and end in a state in which genes A and B are fully expressed, and the gene C is partly expressed (the red circle).

The wave transition times are then shown in a graph showing the change in promoter activity for each of the genes over time. Initially the promoter activity for each of the genes A, B and C is at a low steady state. First, the promoter activity for the gene A rises to a high steady state, with wave transition time $T_A$. As the expression of gene A promotes the expressions of genes B and C, both then rise to a higher steady state, with gene B having a wave transition time of $T_B$ and gene C with a later wave transition time of $T_{C,1}$, simply due to gene C reacting to being promoted by the expression of gene A more slowly than gene B. However, at time $T_{Val,C,1}$, the expression of gene B is sufficient that it begins to inhibit the expression of gene C. This leads to a second wave transition time $T_{C,2}$ for gene C, when its promoter activity moves from the high steady state to a medium steady state, as gene C is being both promoted by the expression of gene A, and inhibited by the expression of gene B.

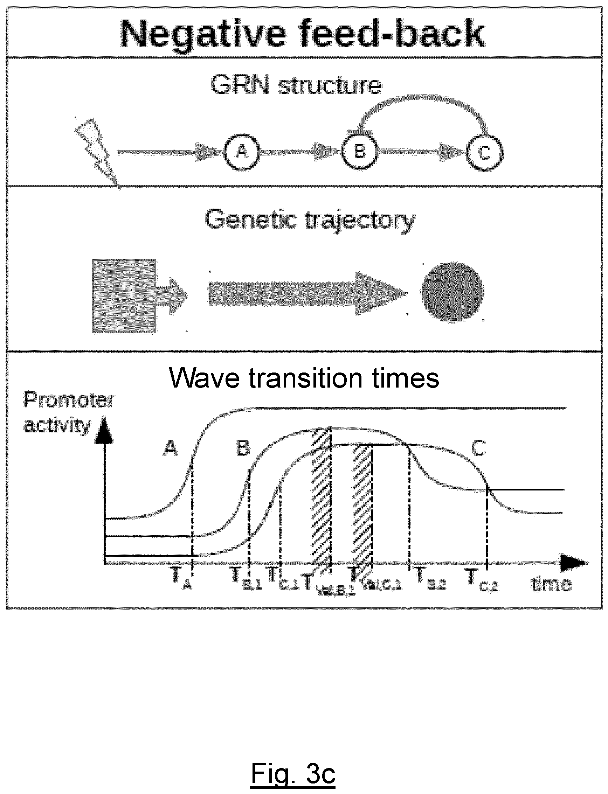

Figure 3c shows a negative feedback GRN structure, in which gene A promotes gene B, gene B promotes gene C, and gene C then inhibits gene B. This behaves similarly to the incoherent feed-forward loop GRN structure of Figure 3b, except that the promoter activity of gene B moves from a low steady state, to a high steady state, to a medium steady state, due to the inhibiting effect on it of gene C. Further, while the promoter activity of gene C again ends in a medium steady state, it is due to gene C inhibiting gene B, so reducing the promoting effect gene B has on gene C, rather than because gene B itself inhibits gene C.

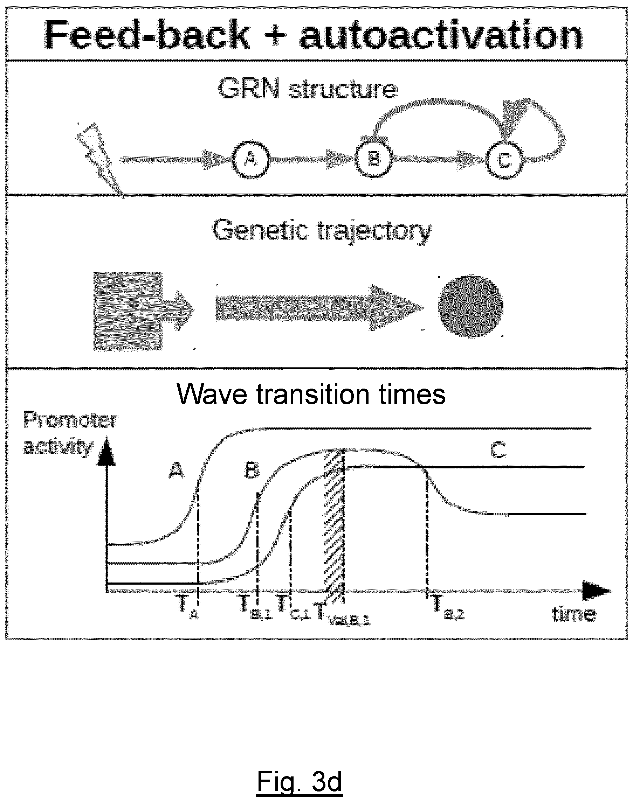

Figure 3d shows a feedback and auto-activation GRN structure. This is the same as the negative feedback GRN structure of Figure 3c, except that the expression of gene C also has a promoting effect on itself. This means that, while its expression is initially caused by gene B, the subsequent inhibition of gene B does not reduce its expression, as it continues to promote its own expression. This results in gene C moving only from a low steady state to a high steady state, with only a single wave transition time.

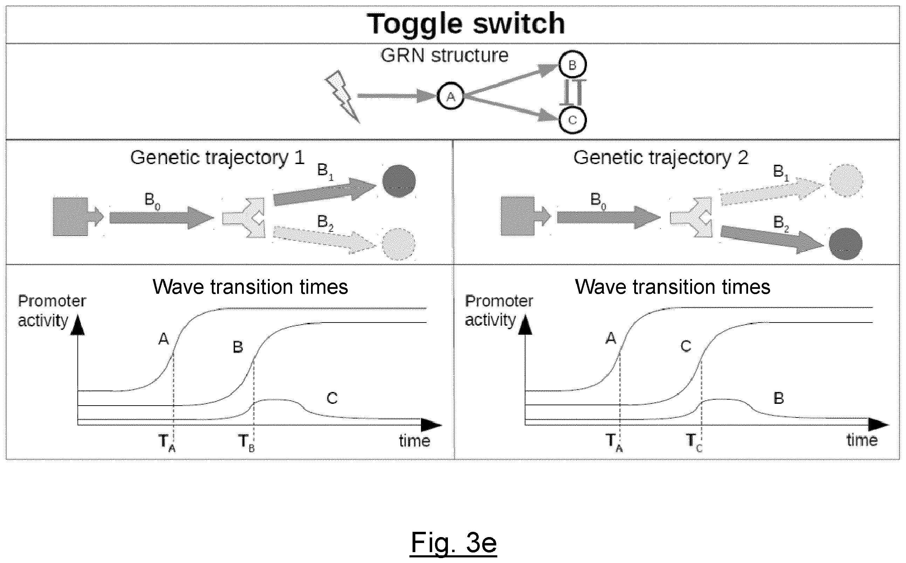

Figure 3e shows a toggle switch GRN structure. In this case, a stimulus promotes gene A, with in turn promotes gene B and gene C. However, gene B inhibits gene C, and gene C inhibits gene B.

Unlike for the above GRN structures, in this case the corresponding genetic trajectory does not end with a single steady state. Instead, there are two possible genetic trajectories, with corresponding graphs of promoter activity. In the first possibility, the expression of gene B successfully inhibits gene C, so genes A and B move from the low steady state to the high steady state with wave transition times $T_A$ and $T_B$, but while the expression of gene C briefly rises, it does not reach a higher steady state and returns to the low steady state, without having any wave transition time. In the second possibility, the expression of gene C successfully inhibits gene B, so genes A and C move from the low steady state to the high steady state with wave transition times $T_A$ and Tc, and gene B returns to the low steady state without having any wave transition time.

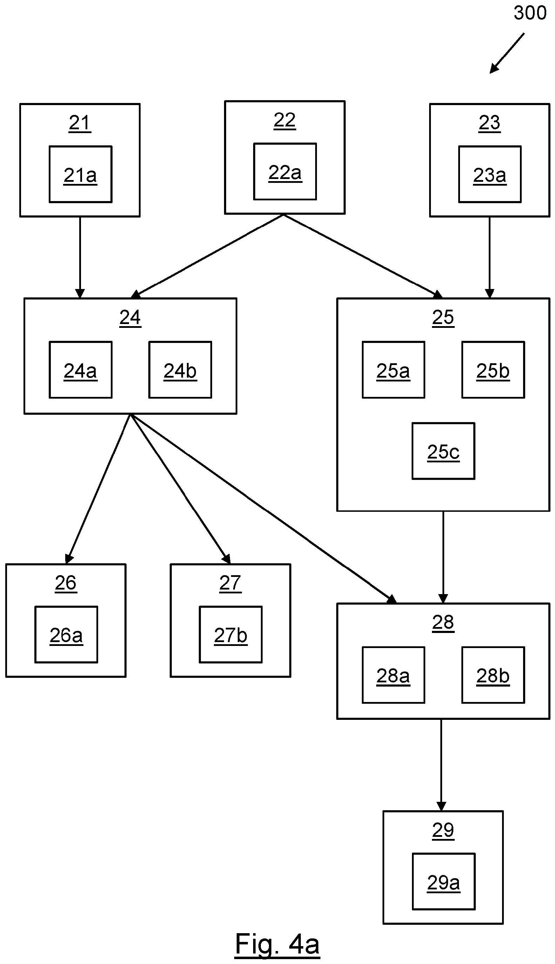

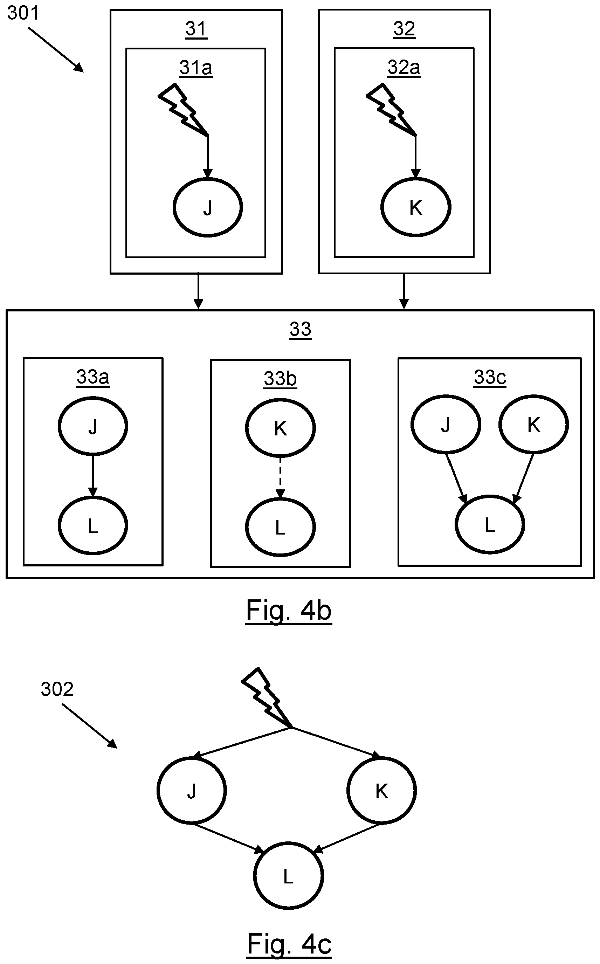

Figures 4a and 4b are diagrams of exemplary generic networks. Figure 4a shows a generic network 300, which comprises sets 21 to 29 of gene interactions. For example, set 29 contains a single gene interaction 21a, while set 25 contains three gene interactions 25a, 25b, 25c. The generic network 300 further comprises dependencies between the sets of gene interactions, as shown by the arrows in Figure 4a. For example, set 24 is dependent upon set 21 and set 22, set 25 is dependent upon set 22 and 23, and set 29 is dependent upon set 28 only.

Figure 4b shows a simpler generic network 301, but in which the content of the gene interactions is shown. The generic network 301 comprises sets 31 to 33 of gene interactions. As can be seen, a gene interaction has one or more regulators (shown at the top of each gene interaction), connected by arrows to one or more targets (shown at the bottom of each gene interaction). The regulators are genes or stimulus, and the targets are genes. The arrows between the regulators and the targets indicate how the regulators affect the targets. For example, in the gene interaction 31a of set 31, the gene J is promoted by the stimulus, as indicated by the solid arrow. Similarly, in the gene interaction 32a of set 32, the gene K is promoted by the stimulus. The set 33 contains three gene interactions, gene interaction 33a, in which the gene J promotes the gene L; gene interaction 33b, in which the gene K inhibits the gene L; and gene interaction 33c, in which the gene J and gene K together promote the gene L.

As can be seen, the target of each of the gene interactions 33a, 33b, 33c of the set 33 is the gene L. Further, the regulators of the gene interactions 33a, 33b, 33c are either gene J, gene K, or both. Thus, the gene interactions 33a, 33b, 33c represent different possible interactions between genes that could led to the genes J and/or K affecting the behaviour of the gene L (whether by promotion or inhibition). Further, the regulators of the set 33 are the same as the (collective) targets of the sets 31 and 32, i.e. the genes J and K. Thus, where a second set of gene interactions of a generic network is dependent upon a first set of gene interactions, this indicates that first set contains gene interactions that are possible behaviours of genes in a GRN, and the second set contains gene interactions that are possible subsequent behaviours of genes, i.e. triggered by one of the behaviours in the first set.

It will be appreciated that a generic network may represent more complicated dependencies between sets of gene interactions, where the regulators and targets of the gene interactions of the sets allow. For example, a third set may be dependent upon a second set, and the second set dependent upon a first set. As long as the targets of the first set are shared by the regulators of the third set, the third set may also be dependent upon the first set, i.e. upon its "grandparent", so skipping a generation.

Figure 4c shows the GRN 302 that may be underlying the generic network 301, in which the (single) stimulus promotes genes J and K, which in turn promote gene L. This GRN comes from the combination of the gene interactions 31a, 32a and 33c.

Returning to the flowchart of Figure 2, as mentioned above, in step 203 of the method of Figure 2, gene parameters are estimated. The gene parameters that are estimated are parameters such as RNA and protein degradation and synthesis rates, which can be determined using any appropriate techniques, of which various will be well-known to the skilled person. For example, to estimate RNA degradation rates, the change over time of RNA molecule count after an inhibition of transcription of RNA can be measured from the multi-omic data. This can show an exponential decay of the RNA, allowing an estimation of the half-life of the RNA which gives the degradation rate. Equivalent techniques are known for protein degradation and synthetic rates using SILAC technology, as described in Schwanhäusser, B., Busse, D., Li, N. et al. Global quantification of mammalian gene expression control. Nature 473, 337-342 (2011).

In step 204, genetic trajectories are identified from the multi-omic data. As mentioned above, a gene trajectory describes the change in gene behaviour over time, but without any indication of the interactions between the genes that cause the behaviour. As discussed above, the data that is available shows a snapshot of the behaviour of different cells at different moments in time, while for a genetic trajectory, the behaviour of a single cell over time is required. To allow the latter to be determined from the former, the "ergodic assumption" is used. The ergodic assumption states that measuring multiple cells at one time is equivalent to measuring a single cell at multiple times. Thus, the data can be considered to show how a single cell behaves at different times. However, the problem is then to order the snapshots of behaviour in time. This is discussed below.

In step 205, the cells and/or genes to be used later in the method are selected. These are used when identifying the genetic trajectories (i.e. the genetic trajectories relevant to the selected cells/genes are identified), and are in turn the process of identifying genetic trajectories helped to refine which cells/genes to select, feeding back into the identification of the genetic trajectories, and so on.

The results from the above steps are used to identify wave transition times in step 206.

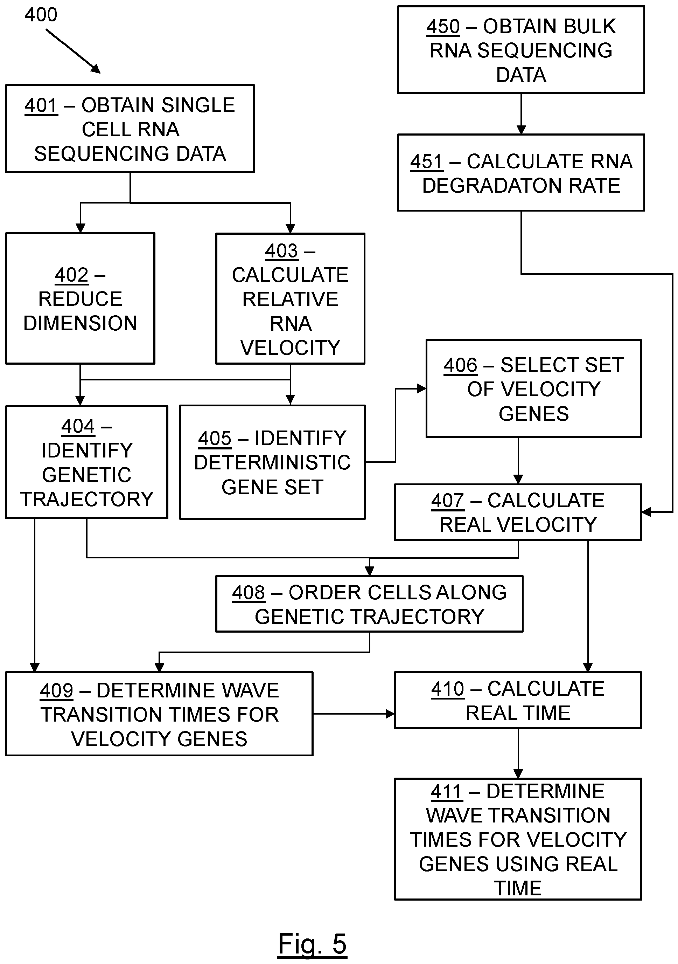

Figure 5 is a flowchart 400 showing in more detail the processes underlying of the above steps. In a first step, single cell RNA sequencing data is obtained from the multi-omic data (step 401). Dimension reduction is performed on this data (step 402), to allow it to be visualized in the genetic space. This can be done using any appropriate method, for example UMAP (uniform manifold approximation and projection) or PCA (principal component analysis).

In parallel, bulk RNA sequencing (transcription inhibition) data is obtained (step 450), and is used to determine RNA degradation rate $d_0$ for different genes (step 451). In a first step, a relative and approximative RNA velocity is then calculated from the single cell RNA sequencing data using standard algorithms such as scVelo (Bergen et al. Nature Biotech, 2020), that only use the spliced (S) and unspliced (U) RNA information (step 403). This RNA velocity is fundamentally relative, i.e. it does not correspond to a real derivative value of RNA quantity per time unit, because RNA degradation rate is not used by existing algorithm and RNA degradation rates are required to compute the "REAL" RNA velocity, as described in step 407.

Using the dimension reduced data and the relative RNA velocities, a genetic trajectory of interest is identified (step 404) using any suitable method, of which various standard methods will be known to the skilled person, for example bioinformatic tools such as clustering, density plot, DE (differential expression analysis), GSEA (gene set enrichment analysis), GO (gene ontology analysis) and the like. In this way, "groups" of cells with associated biological functions can be identified. In addition, a deterministic gene set is identified (step 405); this is a set of genes whose expression behaviour is primarily deterministic (noise-free) rather than stochastic ("bursty"). A gene set of "velocity" genes is then created (step 406), which consists of deterministic genes with known $d_0$.

For each velocity gene (i.e. each gene of the set of velocity genes), a "REAL" velocity is calculated (step 407). This is done, for each gene, using the following differential equation:

$$RNA\ velocity = \frac{dS}{dt} = S_p \cdot U(t) - d_0 \cdot S(t)$$

in which S is spliced RNA, U is unspliced RNA, $S_p$ is the splicing rate, t is time, and $d_0$ is RNA degradation rate. $d_0$ is obtained using transcription inhibition experiments, and $S_p$ can be estimated as follows:

$$S_p = d_0 \left(\frac{Smean}{Umean}\right)$$

in which Smean and Umean are respectively the mean value of S and U among all cells.

The REAL velocity is an improvement of the relative RNA velocity initially calculated. The previous steps can then be repeated, improving the genetic trajectory of interest identified, until no more improvements are made, i.e. the genetic trajectory obtained using the improves REAL velocities is the same as the genetic trajectory previously obtained. This will be when the minimum set of genes required to generate the cell behaviour of interest have been selected.

Once the genetic trajectory of interest has been finalised, the cells are time-ordered along the genetic trajectory (step 408). This is done by matching the observed expression of their relevant genes to the genetic trajectory. However, it should be noted that this does not give a specific time at which a single cell would have that particular behaviour, so is not indicative of how quickly the cell changes from one behaviour to the next, but only gives an ordering in time.

Wave transition times for velocity genes are then determined from the ordering (step 409), and "REAL" times for the cells are calculated (step 410), using the REAL velocities. The REAL times represent the times at which the cells in their state occur in the life cycle of the cell, so are "actual" times, not merely indicative of a time ordering. Wave transition times for velocity genes are then determined from the REAL times (step 411). Each of these steps is now described in detail.

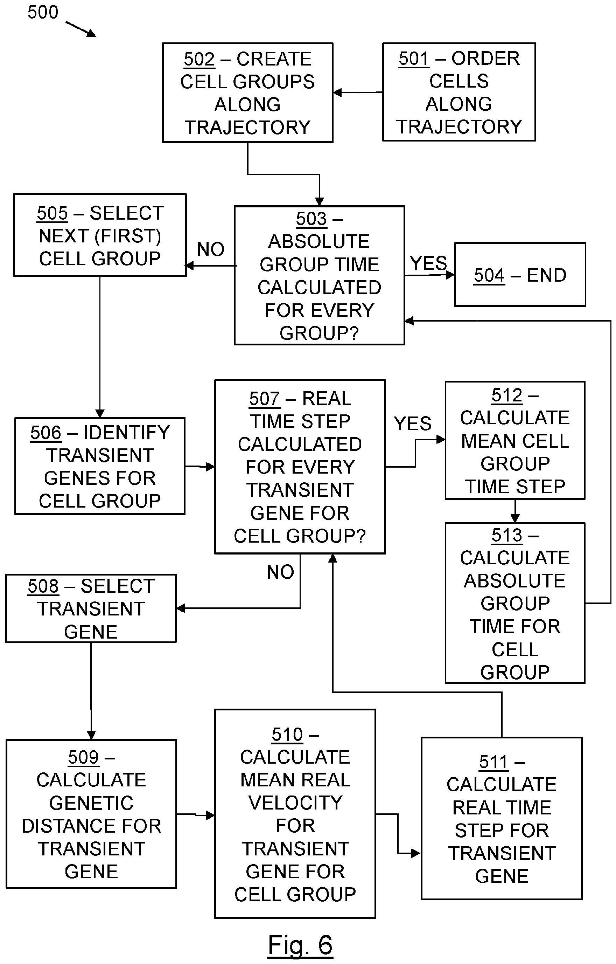

Figure 6 is a flowchart 500 showing the calculation of absolute group times for the cell groups, from which the REAL times for the cells are then calculated. As above in step 408, the cells are time-ordered along the genetic trajectory (step 501). The branches of the genetic trajectory are then considered separately. For a selected branch, cell groups are created, which are groups of cells which adjacent to each other (step 502). If an absolute group time has been calculated for every cell group (step 503), the calculation is finished (step 504). If not the next (or first) cell group on the branch is selected (step 505). Transient genes of the cell group are identified (step 506), which are genes whose expression changes from one state to another within the cell group, i.e. those whose gene quantity value passes from one steady state to another steady state.

If a REAL time step $dt_{gene}$ has not been calculated for transient gene in the cell group (step 507), the next transient gene is selected (step 508). The genetic distances $dD_{gene}$ of the transient gene from the previous cell group (if there is one) is then determined (step 509). This can be done using any appropriate method, including methods well-known to the skilled person, with the genetic distance being indicative of the difference between the state of the cell and the state of a reference cell. The mean REAL velocity $V_{gene}$ for transient gene in the cell group is then computed (step 510) considering only cells for which the genetic distance and REAL velocity are coherent.

The REAL time step $dt_{gene}$ for the transient gene is then calculated (step 511) from $dD_{gene}$ and $V_{gene}$ as follow:

$$dt_{gene} = \frac{dD_{gene}}{V_{gene}}$$

Step 507 is then returned to, and any further REAL time step for transient gene are calculated. Once a REAL time step has been calculated for every translation gene of the cell group, the cell group time step $dt_{group}$ is then computed (step 512) as the mean of the gene times $dt_{gene}$ for the cell group.

The absolute group time for the cell group is then calculated iteratively (step 513), by adding the cell group time step for the previous cell group to the absolute cell group time for the previous cell group:

$$t_{group(n+1)} = t_{group(n)} + dt_{group(n)}$$

Step 503 is then returned to, with any further absolute group times for cell groups being calculated as required.

Once all the absolute group times for cell groups have been calculated, the REAL times for the cells are calculated as the averages of the absolute cell group times for the cell groups in which the cells are present. Thus, if a cell is only present in one cell group, its REAL time is the absolute cell group time of the cell group in which it occurs. However, cell groups can desirably overlap, when there is more than one cell group a cell is included in. In this case, the REAL time for the cell is the average of the absolute cell group times of the cell groups in which it occurs.

As in step 411 above, wave transition times for velocity genes are then determined from the REAL times of the cells. Further, the wave transition times for the other important genes in the genetic trajectory of interest are required. In the present embodiment this is done by identifying the differentially expressed genes in the genetic trajectory along the REAL time and estimating the inflexion time point, though it will be appreciated that other methods are possible. The differentially expressed genes are those which exhibit significantly different behaviour at different times during the genetic trajectory, and they are used to reduce the number of genes that need to be considered to a manageable number, for example from tens of thousands of genes to less than one hundred. Using this, the wave transition times for the genes can be determined using techniques known to the skilled person, see for example Arnaud Bonnaffoux. Inférence de réseaux de régulation de gènes à partir de données dynamiques multi-échelles. Bio-Informatique, Biologie Systémique [q-bio.QM]. Universit6 de Lyon, 2018.

As in step 207 of Figure 2 above, the wave transition times are then used to construct the generic network.

Essentially, this is done by associating the wave transition times with interactions between genes that could cause those wave transition times, i.e. the structures and corresponding behaviours examples of which are shown in Figures 3a to 3e.

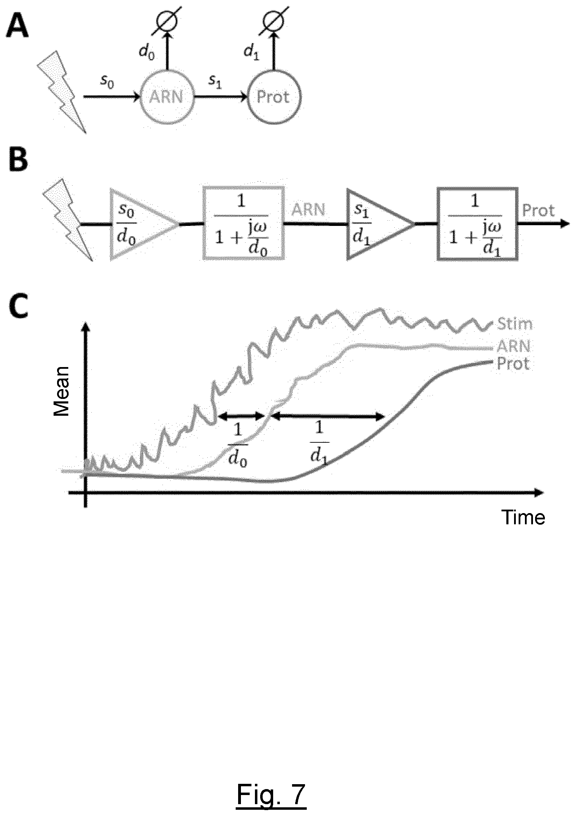

In practice, this is performed using the estimated RNA and protein degradation rates, which can be measured, or otherwise obtained (e.g. from the results of experiments in the scientific literature). As shown schematically in Figure 7, if a gene A promotes a gene B, then the time delay (in REAL time) between the RNA wave transition times for the genes should be:

$$\frac{1}{d_{1A}} + \frac{1}{d_{0B}}$$

where $d_{1A}$ is the protein degradation rate of gene A, and $d_{0B}$ is the RNA degradation rate of gene B.

The gene interactions making up a set of gene interactions in the generic network can then be taken to be all possible interactions between regulators and targets i->j satisfying:

$$\left| (T_j - T_i) - \left( \frac{1}{d_{1A}} + \frac{1}{d_{0B}} \right) \right| < \delta$$

where $T_A$ is the wave transition time for gene A, and $\delta$ is an acceptable error. A typical acceptable $\delta$ is 5 hours.

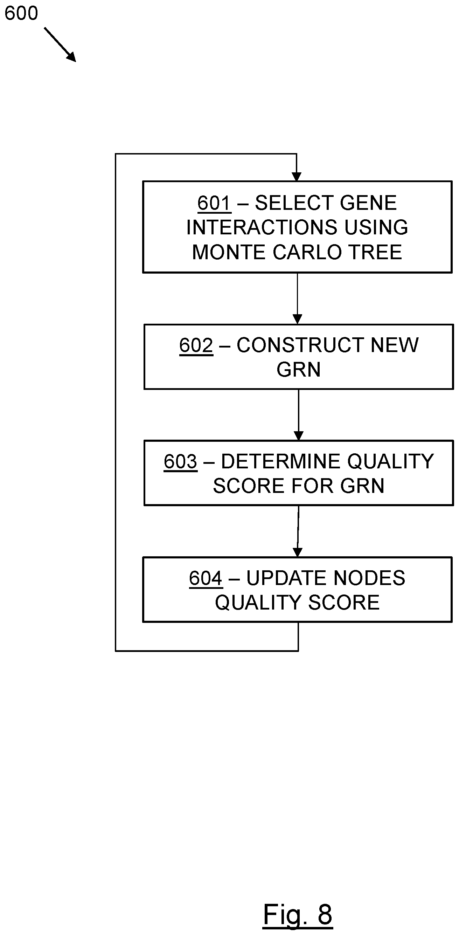

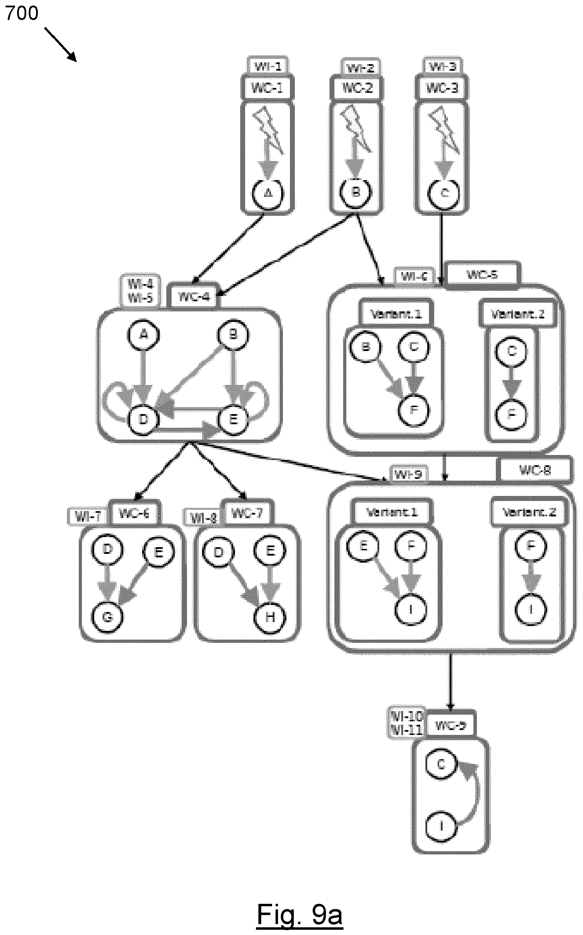

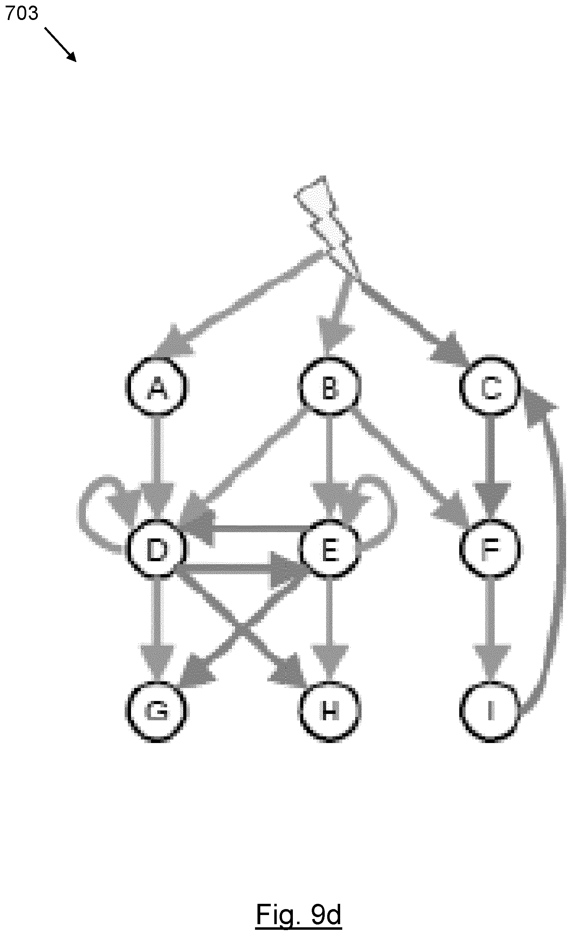

The construction of a GRN from a generic network is now described with reference to Figures 8 to 9d. Figure 9a shows an exemplary generic network 700, which is another example of a generic network like the generic network 300 of Figure 4a and the generic network 301 of Figure 4b.

Figure 8 is a flowchart 600, showing the overall method. As can be seen, it is an iterative method, involving the construction of successive GRNs. First, the gene interactions from the generic network to use to construct the GRN are selected, using a Monte Carlo tree search (step 601). However, it will be appreciated that in other embodiments, other methods could be used to try to find the optimal GRN, for example machine learning methods.

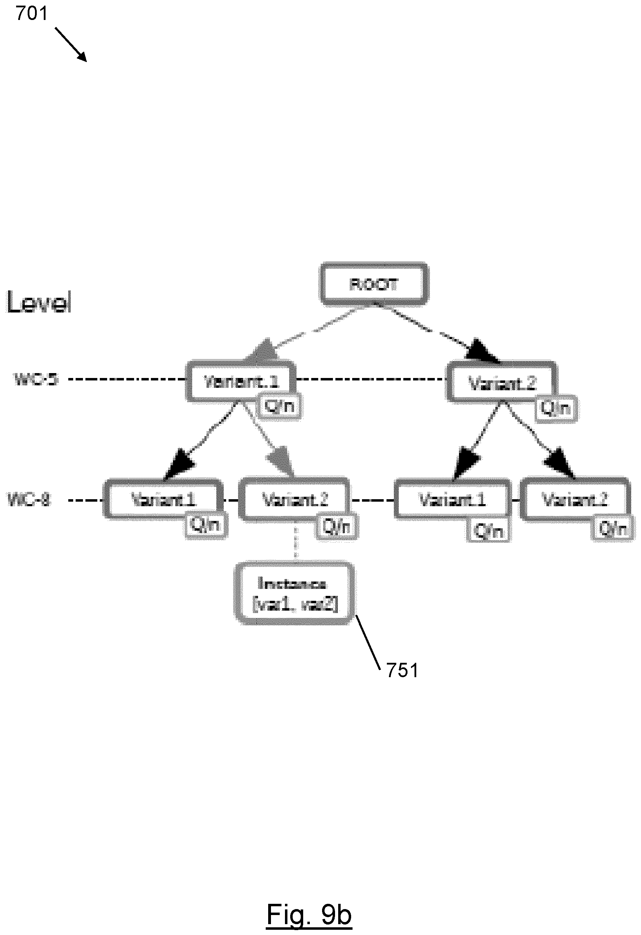

The Monte Carlo tree 701 (or a part thereof) is shown schematically in Figure 9b, in which the Monte Carlo tree 701 has different levels, each level having nodes corresponding to different variants (i.e. gene interactions in a set) that can be selected. Each node has associated with it a quality score Q and a number of visits n. The instance 751 corresponds the selection of the gene interactions shown in red (Variant 1 and Variant 2), with the Monte Carlo Tree search at each interaction selecting an instance corresponding to a selection of gene interactions, based on the quality scores Q, number of visits n, and an exploration coefficient.

The selected gene interactions are then used to construct a new GRN (step 602), as explained in detail below. A quality score is then determined for the newly constructed GRN (step 603), again as discussed below. The nodes of the Monte Carlo tree 701 for the gene interactions used to construct the newly constructed GRN are then updated with the quality score for the newly constructed GRN, and number of visits for each node is also updated.

The quality score for the newly constructed GRN is then used to update the quality score Q for each selected nodes (step 604), and the process is repeated as many times as appropriate. For example, the iterative process can be stopped when every combination of node has been tested, though in practice this is likely not to be computationally tractable. Alternatively, the process may be stopped when the overall quality scores of GRNs appears to have reach a global maximum (e.g. the newly constructed GRNs systematically have a lower quality score), or by any other desired method.

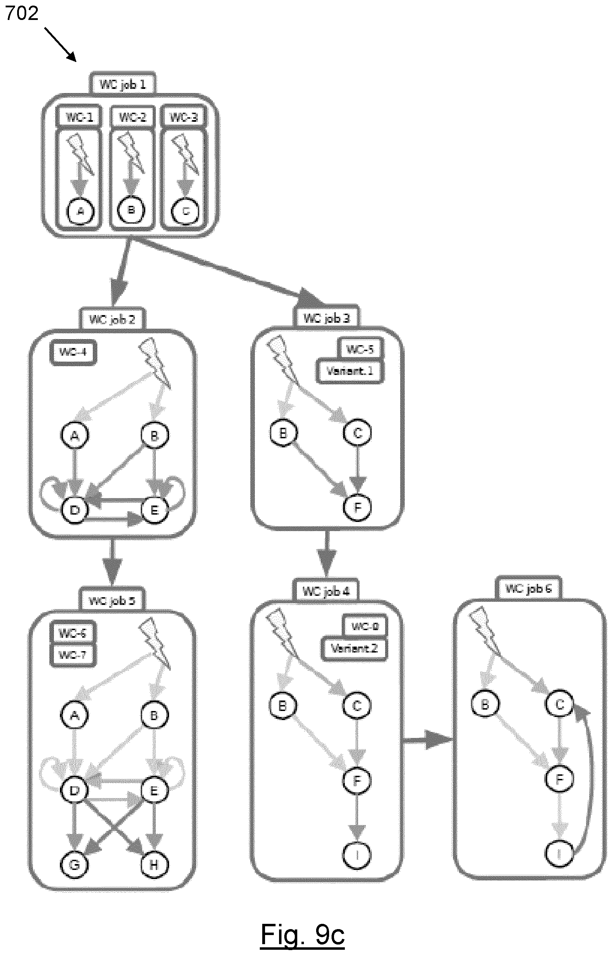

Figure 9c shows successive wave calibration jobs performed in order to construct the GRN in step 602. As can be seen, the wave calibration jobs build the GRN by successively adding the gene interactions, with the jobs branching where appropriate, i.e. when there are multiple gene interactions dependent from the GRN as so far constructed. At each stage, the wave behaviour of the GRN (or relevant constructed branch) is simulated. This is done using any suitable method, for example that described in Bonnaffoux, A., Herbach, U., Richard, A. et al. WASABI: a dynamic iterative framework for gene regulatory network inference. BMC Bioinformatics 20, 220 (2019). Calibration is then performed using an optimisation algorithm to find interaction parameter values for the newly added genes interactions that minimise a fit distance between the simulated behaviour and the measured behaviour from the multi-omic data. (The interaction parameter values represent the strength of an interaction between two genes.) Only the newly added genes interaction are calibrated, as the parent gene interactions have already been calibrated, and do not need to be re-calibrated.

Once this process is complete, the GRN 703 has been constructed, along with all the interaction parameters calibrated to fit the multi-omic data.

To determine the quality score for the GRN 703, a distance metric called Kantorovitch distance, that can be estimated for example with the sinkhorn algorithm (Marco Cuturi, Sinkhorn Distances: Lightspeed Computation of Optimal Transport, NeurIPS 2013), can be used compare the simulated behaviour for the GRN 703 with the measured behaviour from the multi-omic data. However, it will be appreciated that various other methods of determining a quality score will be apparent to the skilled person.

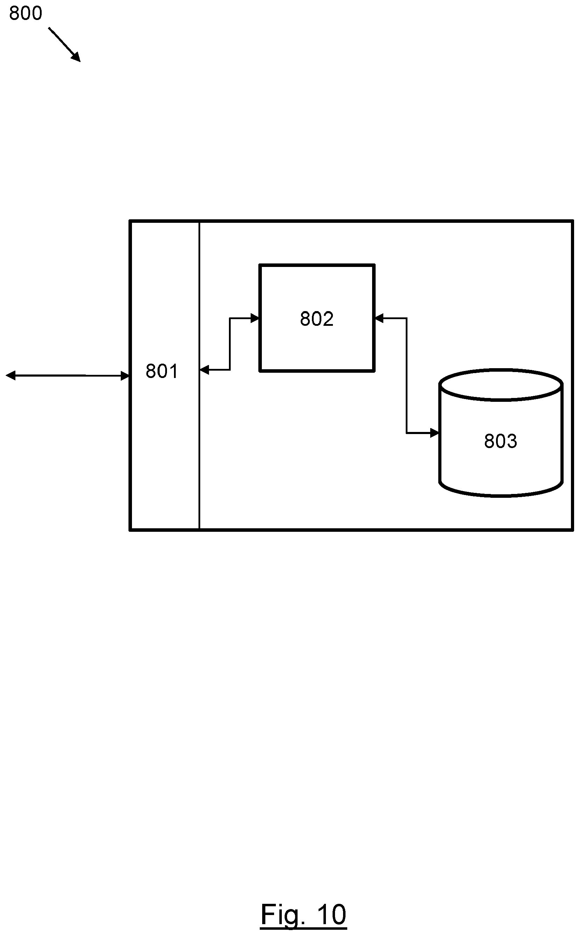

Embodiments of the invention include the methods described above performed on a computing device, such as the computing device 800 shown in Figure 10. The computing device 800 comprises a data interface 801, through which data can be sent or received, for example over a network, and in particular via which the multi-omic data can be received. The computing device 800 further comprises a processor 802 in communication with the data interface 801, and memory 803 in communication with the processor 802. In this way, the computing device 800 can receive data, such as image data, video data, or various data structures, via the data interface 801, and the processor 802 can store the received data in the memory 803, and process it so as to perform the methods described herein, including processing image data and/or generating images.

Each device, module, component, machine or function as described in relation to any of the embodiments described herein may comprise a processor and/or processing system or may be comprised in apparatus comprising a processor and/or processing system. One or more aspects of the embodiments described herein comprise processes performed by apparatus. In some embodiments, the apparatus comprises one or more processing systems or processors configured to carry out these processes. In this regard, embodiments may be implemented at least in part by computer software stored in (non-transitory) memory and executable by the processor, or by hardware, or by a combination of tangibly stored software and hardware (and tangibly stored firmware). Embodiments also extend to computer programs, particularly computer programs on or in a carrier, adapted for putting the above described embodiments into practice. The program may be in the form of non-transitory source code, object code, or in any other non-transitory form suitable for use in the implementation of the processes. The carrier may be any entity or device capable of carrying the program, such as a RAM, a ROM, or an optical memory device, etc.

While the present invention has been described and illustrated with reference to particular embodiments, it will be appreciated by those of ordinary skill in the art that the invention lends itself to many different variations not specifically illustrated herein. By way of example only, certain possible variations will now be described.

Where in the foregoing description, integers or elements are mentioned which have known, obvious or foreseeable equivalents, then such equivalents are herein incorporated as if individually set forth. Reference should be made to the claims for determining the true scope of the present invention, which should be construed so as to encompass any such equivalents. It will also be appreciated by the reader that integers or features of the invention that are described as preferable, advantageous, convenient or the like are optional and do not limit the scope of the independent claims. Moreover, it is to be understood that such optional integers or features, whilst of possible benefit in some embodiments of the invention, may not be desirable, and may therefore be absent, in other embodiments.

**Claims**

1. A computer-implemented method of constructing a gene regulatory network for a set of cells, wherein a gene regulatory network is a network showing interactions between a plurality of genes, the method comprising the steps of:

   receiving data including a set of gene quantity values, wherein each gene quantity value is associated with a combination of a cell of the set of cells and a gene of the cell, and wherein each gene quantity value is indicative of a measurement of a gene quantity for the gene, wherein a gene quantity is a quantity of a product resulting from the expression of the gene;
   determining a plurality of sets of the gene quantity values, wherein each gene quantity value of a set of gene quantity values is associated with a different gene;
   for each of the plurality of sets of gene quantity values, determining a time value for the set of gene quantity values from the gene quantity values of the set of gene quantity values;
   for each of a plurality of genes, determining the level of the gene quantity value for the gene over time from the determined times of the sets of gene quantity values containing the gene quantity value;
   for each of a plurality of genes, determining a wave transition time for the gene quantity value for the gene from the determined the level of the gene quantity value for the gene over time, wherein the wave transition time is indicative of the time at which a gene quantity value passes from one steady state to another steady state;
   determining a set of interactions between the genes using the wave transition times for the plurality of genes; and
   constructing a gene regulatory network from the determined set of interactions between the genes.

2. A method as claimed in claim 1, wherein a gene quantity of the gene quantities is one of:

   an RNA count associated with the expression of the gene; and
   a protein count associated with the expression of the gene.

3. A method as claimed in claim 1 or 2, wherein the time value for each set of gene quantity values is determined by:

> determining a genetic distance value indicative of the difference between the gene quantity values of the set of gene quantity values and corresponding gene quantity values of a reference cell;
> determining an RNA velocity value indicative of the rate of change of the gene quantity values of the set of gene quantity values in a reference cell; and
> determining the time value from the genetic distance value and the RNA velocity value.

4. A method as claimed in claim 3, wherein the gene quantity values are indicative of a measurement a spliced RNA count and an unspliced RNA count for the gene, and wherein the RNA velocity is calculated using the spliced RNA count and the unspliced RNA count.

5. A method as claimed in claim 4, wherein the method further comprises the step of receiving, for each combination of a cell and a gene, an RNA degradation rate, where the RNA degradation rate is indicative of the degradation rate of the spliced RNA, and wherein the RNA velocity is calculated using the spliced RNA count, the unspliced RNA count and the RNA degradation rate.

6. A method as claimed in any preceding claim, further comprising the step of obtaining one or more genetic trajectories for cells of the set of cells, where a genetic trajectory for a cell is indicative of changes in steady states of the expression of a set of genes of the cell over a period of time, and wherein one or more wave transition times are determined using the one or more genetic trajectories.

7. A method as claim in 6, wherein the method further comprises the step of determining a time ordering for a plurality of cells of the set of cells using the genetic quantity values for the cell and their associated genes, where the ordering is in accordance with the ordering of the expressions of the genes in the one or more genetic trajectories.

8. A method as claimed in claim 7, further comprises the steps of:

> determining a cell group, wherein a cell group is a subset of the plurality of cells, and wherein each cell in the cell group is adjacent to at least one other cell in the time ordering for the plurality of cells;
> identifying one or more transient genes of the cell group, wherein a transient gene is a gene whose gene quantity value passes from one steady state to another steady state in the cells of the cell group;
> determining an overall genetic distance value for the one or more transient genes of the cell group;
> determining an overall RNA velocity value for the one or more transient genes of the cell group;
> determining an overall time value for the cell group from the overall genetic distance value and the overall RNA velocity value; and
> using the overall time value for the cell group to determine the wave transition times.

9. A method as claimed in claim 8, wherein an overall time value for each of a plurality of cell groups is determined, and wherein the overall genetic distance value for a first cell group is indicative of the difference between the gene quantity values of the cells of the first cell group and the corresponding gene quantity values of a second cell group.

10. A method as claimed in any preceding claim, wherein at least one interaction of the set of interactions between the genes is included in the set of interactions if the interaction is between a first gene and a second gene, and the difference between a wave transition time for the first gene and between a wave transition time for the second gene is below a threshold.

11. A computer-implemented method of constructing a gene regulatory network for a set of cells, wherein a gene regulatory network is a network showing interactions between a plurality of genes, the method comprising the steps of:

> receiving data including a set of gene quantity values, wherein each gene quantity value is associated with a combination of a cell of the set of cells and a gene of the cell, and wherein each gene quantity value is indicative of a measurement of a gene quantity for the gene, wherein a gene quantity is a quantity of a product resulting from the expression of the gene;
> determining from the set of gene quantity values a plurality of gene interactions, wherein a gene interaction comprises one of more regulators which are indicative of a stimulus or a gene of the set of genes, one or more targets which are indicative of genes of the set of genes, and a set of relationships which are indicative of promotion or inhibition of a target of the gene interaction by a regulator of the gene interaction;

determining a network of sets of gene interactions, wherein each gene interaction of a set of gene interactions shares at least one regulator and at least one target, and wherein a first set of gene interactions is dependent on a second set of gene interactions only if a shared regulator of the first gene interaction is a shared target of the second gene interaction;

constructing the gene regulatory network from the network of sets of gene interactions, by selecting a gene interaction of each set of gene interactions, and combining the selected gene interactions by, for each selected gene interaction, and for each selected gene interaction on which the first selected gene interaction is dependent, matching each regulator of the first selected gene interaction with a corresponding target of a selected gene interaction on which it is dependent.

12. A method as claimed in claim 11, further comprising the steps of:

calculating a quality score for the constructed the gene regulatory network;
comparing the quality score with a calculated quality score for a previously constructed gene regulatory network; and
constructing a further gene regulatory network from the network of sets of gene interactions, wherein the gene interactions are selected from each set of gene interactions based on the calculated quality scores.

13. A method as claimed in any of claims 1 to 10, further comprising the method of any of claims 11 or 12.

14. A method as claimed in any preceding claim, further comprising the step of performing measurements on the set of cells to determine the set of gene quantity values for the set of cells.

15. A computer program product comprising machine-readable instructions that, when executed on a computing device comprising a processor and memory, cause the computing device to perform the steps of the methods of any of claims 1 to 13.

100

101

A    B    C

D    E    F

G    H    I

Fig. 1

200

201 – RECEIVE MULTI-OMIC DATA

202 – PRE-PROCESS DATA

203 – ESTIMATE GENE PARAMETERS

204 – IDENTIFY GENETIC TRAJECTORIES

205 – SELECT CELLS AND/ OR GENES

206 – IDENTIFY WAVE TRANISITION TIMES

207 – CONSTRUCT GENERIC GRN

208 – CONSTRUCT GRN

Fig. 2

**Cascade**

GRN structure

Genetic trajectory

Wave transition times

Fig. 3a

Fig. 3b

Fig. 3c

Fig. 3d

**Toggle switch**

GRN structure

Genetic trajectory 1

$B_1$
$B_0$
$B_2$

Genetic trajectory 2

$B_1$
$B_0$
$B_2$

Wave transition times

Promoter activity

A
B
C

$T_A$    $T_B$    time

Wave transition times

Promoter activity

A
C
B

$T_A$    $T_C$    time

<u>Fig. 3e</u>

EP 4 506 947 A1

Fig. 4a

301

31

31a

J

32

32a

K

33

33a

J

L

33b

K

L

33c

J     K

L

Fig. 4b

302

J          K

L

Fig. 4c

400

401 – OBTAIN SINGLE CELL RNA SEQUENCING DATA

450 – OBTAIN BULK RNA SEQUENCING DATA

451 – CALCULATE RNA DEGRADATON RATE

402 – REDUCE DIMENSION

403 – CALCULATE RELATIVE RNA VELOCITY

406 – SELECT SET OF VELOCITY GENES

404 – IDENTIFY GENETIC TRAJECTORY

405 – IDENTIFY DETERMINISTIC GENE SET

407 – CALCULATE REAL VELOCITY

408 – ORDER CELLS ALONG GENETIC TRAJECTORY

409 – DETERMINE WAVE TRANSITION TIMES FOR VELOCITY GENES

410 – CALCULATE REAL TIME

411 – DETERMINE WAVE TRANSITION TIMES FOR VELOCITY GENES USING REAL TIME

Fig. 5

500

Fig. 6

**A**

**B**

$$\frac{s_0}{d_0} \qquad \frac{1}{1+\dfrac{\mathrm{j}\omega}{d_0}} \qquad \text{ARN} \qquad \frac{s_1}{d_1} \qquad \frac{1}{1+\dfrac{\mathrm{j}\omega}{d_1}} \qquad \text{Prot}$$

**C**

Fig. 7

600

601 – SELECT GENE INTERACTIONS USING MONTE CARLO TREE

602 – CONSTRUCT NEW GRN

603 – DETERMINE QUALITY SCORE FOR GRN

604 – UPDATE NODES QUALITY SCORE

Fig. 8

700

<u>Fig. 9a</u>

701

Fig. 9b

702

Fig. 9c

703

Fig. 9d

800

Fig. 10

Europäisches
Patentamt

European
Patent Office

Office européen
des brevets

## EUROPEAN SEARCH REPORT

Application Number

**EP 23 30 6367**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Singh Rohit ET AL: "Causal gene regulatory analysis with RNA velocity reveals an interplay between slow and fast transcription factors", bioRxiv, 28 February 2023 (2023-02-28), pages 1-21, XP093122041, DOI: 10.1101/2022.10.18.512766 Retrieved from the Internet: URL:https://www.biorxiv.org/content/10.1101/2022.10.18.512766v2.full.pdf [retrieved on 2024-01-22] * p. 1, par. 1 * | 1-10, 13-15 | INV. G16B5/00 G16B25/00 |

-/--

TECHNICAL FIELDS
SEARCHED          (IPC)

G16B

The present search report has been drawn up for all claims

| Place of search | Date of completion of the search | Examiner |
|---|---|---|
| Munich | 25 January 2024 | Mühlbauer, Max |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

Europäisches
Patentamt

European
Patent Office

Office européen
des brevets

# EUROPEAN SEARCH REPORT

Application Number

EP 23 30 6367

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BONNAFFOUX ARNAUD ET AL: "WASABI: a dynamic iterative framework for gene regulatory network inference", BMC BIOINFORMATICS, vol. 20, no. 1, 2 May 2019 (2019-05-02), XP093123116, GB ISSN: 1471-2105, DOI: 10.1186/s12859-019-2798-1 Retrieved from the Internet: URL:http://link.springer.com/article/10.11 86/s12859-019-2798-1/fulltext.html> | 1,2, 6-10, 13-15 | |
| Y | * p. 1, right column, par. 3 p. 2, right column, par. 5 p. 3, Figure 1 and description thereof p. 6, left column, par. 1 p. 11, left column, par. 2 p. 13, left column, section "Methods", par. 2 p. 14, Figure 8 and description thereof p. 16, left column, section "Inflexion estimator for wave time estimation" p. 16, right column, par. 2 p. 17, left column, "Algorithm 1" and detailed description thereof in the paragraphs below p. 17, left column, par. 4 * | 3-5 | |

-----

-/--

TECHNICAL FIELDS
SEARCHED      (IPC)

The present search report has been drawn up for all claims

| Place of search | Date of completion of the search | Examiner |
|---|---|---|
| Munich | 25 January 2024 | Mühlbauer, Max |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GORIN GENNADY ET AL: "RNA velocity unraveled", PLOS COMPUTATIONAL BIOLOGY, vol. 18, no. 9, 12 September 2022 (2022-09-12), page e1010492, XP093123125, US ISSN: 1553-7358, DOI: 10.1371/journal.pcbi.1010492 * p. 6, Figure 2 p. 7, section "Inference" p. 7, Figure 3 and description thereof * | 3-5,8,9 | |
| Y | LIU RUISHAN ET AL: "Dynamical Systems Model of RNA Velocity Improves Inference of Single-cell Trajectory, Pseudo-time and Gene Regulation", JOURNAL OF MOLECULAR BIOLOGY, ACADEMIC PRESS, UNITED KINGDOM, vol. 434, no. 15, 27 April 2022 (2022-04-27), XP087124559, ISSN: 0022-2836, DOI: 10.1016/J.JMB.2022.167606 [retrieved on 2022-04-27] * p. 3, Figure 1 and description thereof * | 3-5 | TECHNICAL FIELDS SEARCHED (IPC) |
| A | PRATAPA ADITYA ET AL: "Benchmarking algorithms for gene regulatory network inference from single-cell transcriptomic data", NATURE METHODS, NATURE PUBLISHING GROUP US, NEW YORK, vol. 17, no. 2, 6 January 2020 (2020-01-06), pages 147-154, XP037006737, ISSN: 1548-7091, DOI: 10.1038/S41592-019-0690-6 [retrieved on 2020-01-06] * the whole document * | 1-10, 13-15 | |

The present search report has been drawn up for all claims

| Place of search | Date of completion of the search | Examiner |
|---|---|---|
| Munich | 25 January 2024 | Mühlbauer, Max |

Europäisches
Patentamt

European
Patent Office

Office européen
des brevets

**Application Number**

EP 23 30 6367

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

**see sheet B**

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

**1-10 (completely); 13-15 (partially)**

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION**

**SHEET B**

The Search Division considers that the present European patent application does not comply with the requirements of unity of invention and relates to several inventions or groups of inventions, namely:

1. claims: 1-10(completely); 13-15(partially)

(i) A computer-implemented method of constructing a gene regulatory network for a set of cells, wherein a gene regulatory network is a network showing interactions between a plurality of genes, the method comprising the steps of:
(ii) receiving data including a set of gene quantity values, wherein each gene quantity value is associated with a combination of a cell of the set of cells and a gene of the cell, and wherein each gene quantity value is indicative of a measurement of a gene quantity for the gene, wherein a gene quantity is a quantity of a product resulting from the expression of the gene;
(iii) determining a set of interactions between the genes
(iv) constructing a gene regulatory network from the determined set of interactions between the genes.
FURTHER COMPRISING
(v) determining a plurality of sets of the gene quantity values,
wherein each gene quantity value of a set of gene quantity values is associated with a different gene;
(vi) for each of the plurality of sets of gene quantity values, determining a time value for the set of gene quantity values from the gene quantity values of the set of gene quantity values;
for each of a plurality of genes, determining the level of the gene quantity value for the gene over time from the determined times of the sets of gene quantity values containing the gene quantity value;
(vii) for each of a plurality of genes, determining a wave transition time for the gene quantity value for the gene from the determined the level of the gene quantity value for the gene over time,
wherein the wave transition time is indicative of the time at which a gene quantity value passes from one steady state to another steady state;
(viii) determining a set of interactions between the genes using the wave transition times for the plurality of genes;
---

2. claims: 11, 12(completely); 13-15(partially)

(i) A computer-implemented method of constructing a gene regulatory network for a set of cells, wherein a gene regulatory network is a network showing interactions between a plurality of genes, the method comprising the steps of:
(ii) receiving data including a set of gene quantity values, wherein each gene quantity value is associated with a combination of a cell of the set of cells and a gene of the cell, and wherein each gene quantity value is indicative of a measurement of a gene quantity for the gene, wherein a

page 1 of 2

Europäisches
Patentamt

European
Patent Office

Office européen
des brevets

**LACK OF UNITY OF INVENTION**
**SHEET B**

Application Number

EP 23 30 6367

The Search Division considers that the present European patent application does not comply with the requirements of unity of invention and relates to several inventions or groups of inventions, namely:

```
gene quantity is a quantity of a product resulting from the
expression of the gene;
(iii) determining from the set of gene quantity values a
plurality of gene interactions
(iv) constructing the gene regulatory network
FURTHER COMPRISING
(V) determining from the set of gene quantity values a
plurality of gene interactions,
 wherein a gene interaction comprises one of more regulators
which are indicative of a stimulus or a gene of the set of
genes, one or more targets which are indicative of genes of
the set of genes, and a set of relationships which are
indicative of promotion or inhibition of a target of the
gene interaction by a regulator of the gene interaction;
 (vi) determining a network of sets of gene interactions,
(vii) using the network of sets of gene interactions to
construct the gene regulatory network
 wherein each gene interaction of a set of gene interactions
shares at least one regulator and at least one target, and
wherein a first set of gene interactions is dependent on a
second set of gene interactions only if a shared regulator
of the first gene interaction is a shared target of the
second gene interaction;
 (viii) selecting a gene interaction of each set of gene
interactions,
(ix) and combining the selected gene interactions by, for
each selected gene interaction, and for each selected gene
interaction on which the first selected gene interaction is
dependent,
(x) matching each regulator of the first selected gene
interaction with a corresponding target of a selected gene
interaction on which it is dependent.
                      ---
```

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SCHWANHÄUSSER, B.** ; **BUSSE, D.** ; **LI, N. et al.** Global quantification of mammalian gene expression control. *Nature*, 2011, vol. 473, 337-342
- **BERGEN et al.** *Nature Biotech*, 2020
- **BONNAFFOUX, A.** ; **HERBACH, U.** ; **RICHARD, A. et al.** WASABI: a dynamic iterative framework for gene regulatory network inference. *BMC Bioinformatics*, 2019, vol. 20, 220